# EUROPEAN PATENT APPLICATION

(11) **EP 3 449 898 A1**
(43) Date of publication of application: **06.03.2019**
(21) Application number: 17789642.0
(22) Date of filing: 27.04.2017
(51) Int. Cl.: A61K 8/36, A61K 8/49, A61Q 1/02, A61Q 1/04, A61Q 3/02, A61Q 5/06, A61Q 5/10

(54) **COSMETIC**

(30) Priority: 28.04.2016 JP 2016091005
(71) Applicant: Kokyu Alcohol Kogyo Co., Ltd., Narita-shi Chiba 287-0225 (JP)
(72) Inventor: ARAHIRA, Nana, Narita-shi Chiba 287-0225 (JP); KAWAI, Kiyotaka, Narita-shi Chiba 287-0225 (JP)
(74) Representative: Nuss, Laurent
(86) International application number: PCT/JP2017/016669
(87) International publication number: WO 2017/188360

(57) **Abstract**

The present invention aims to provide a high quality cosmetic which can maintain a certain color before use regardless of storage conditions or lot. The present invention relates to an oily cosmetic that can control the coloring after its application by comprising a certain amount of a specific C16-18 fatty acid, a process for producing the same, and a method for controlling the coloring thereof.

## Description

### [Technical Field]

The present invention relates to a cosmetic that can control coloring, and a method of controlling the coloring thereof.

### [Background Art]

In makeup cosmetics, conventionally ones that exhibit color or color change after application to the skin are commercially available. In particular, a makeup cosmetic which exhibits discoloration after application to the skin, and a cosmetic having no color or pale color which gradually exhibits color change to a darker one upon application to the skin, thus offering enjoyment of inherent color tone, are commercially available.

For example, Patent Document 1 describes that acidic dyes are generally known to have a tendency of discoloration, and that, by combining water-soluble organic acids such as citric acid, anhydrous citric acid, lactic acid, tartaric acid, malic acid, etc., coloring property and stability over time are improved. In addition, Patent Document 2 describes as follows: regarding fluorescein-based dyes which have been used in oily lipsticks for a long time, such as Red No. 218 (tetrachlorotetrabromofluorescein), Red No. 223 (tetrabromofluorescein) and Orange No. 201 (dibromofluorescein), at the time application, these dyes cause roughness of the lips by ring opening of the lactone ring and dyeing of skin proteins thereof; in addition, at the time of storage, these dyes develop color, resulting in discoloration of the lipstick itself which causes a problem in stability over time; and, these problems have been solved by using a fluorine-based oil agent.

In addition, Patent Document 3 describes a color change composition comprising an active substance and a microencapsulated colorant and a non-encapsulated colorant, which provides a color-change effect upon application to the skin.

Meanwhile, oily cosmetics for the lips blended with an oil-soluble organic acid such as isostearic acid are commercially available; however, these oil-soluble organic acids are blended in a relatively large amount of 10% due to binders, etc., as described in "Collection of Known Technologies in the Field of Cosmetics, 2010 Edition, p.18-19."

### [Citation List]

### [Patent Document]

Patent Document 1: JP A No. H7-206635
Patent Document 2: JP A No. H7-082116
Patent Document 3: JP A No. 2011-519969

### [Non-patent Document]

Non-patent Document 1: Collection of Known Technologies in the Field of Cosmetics, 2010 Edition, Japan Cosmetic Industry Association, Patent Committee, p.18-19.

### [Summary of the Invention]

### [Problems to Be Solved by the Invention]

In oily cosmetics, problems of discoloration of cosmetics before use due to low storage stability, such as discoloration caused by temperature and humidity before use, and differences in the degree of discoloration between lots, are occurring. In addition, highly palatable cosmetic products enjoying color change from colorless or light color before application to the skin to a dark color after application to the skin are expected; therefore, it is desired to provide a high-quality cosmetic which can maintain a certain color before use regardless of storage conditions and lot.

### [Means for Solving the Problems]

In order to solve the above-mentioned problems, the inventors of the present invention have conducted extensive research on ingredients capable of enhancing storage stability and preventing discoloration before application to the skin, and have found that fatty acids such as palmitic acid, isostearic acid and stearic acid can overcome the above-mentioned problems in a stable manner without being affected by changes in the amount of moisture over time, and thus have completed the present invention.

Namely, the present invention relates to the following [1] to [8].
[1] An oily cosmetic comprising a fluorescein dye comprising a lactone moiety, wherein the blending amount of a linear or branched oil-soluble fatty acid having 16-18 carbon atoms is 0.03-2.00%.
[2] The oily cosmetic according to [1], wherein the blending amount of the linear or branched oil-soluble fatty acid having 16-18 carbon atoms is 0.03-0.10%.
[3] The oily cosmetic according to [1] or [2], wherein the linear or branched oil-soluble fatty acid having 16-18 carbon atoms is palmitic acid, isostearic acid or stearic acid.
[4] The oily cosmetic according to any one of [1] to [3], wherein its color changes after application to the lips, skin, hair, or nails.
[5] The oily cosmetic according to any one of [1] to [4], wherein the C.I. number of the fluorescein dye comprising a lactone moiety is C.I. 45410 or C.I. 45380.
[6] The oily cosmetic according to any one of [1] to [5], wherein the oily cosmetic is foundation, lipstick, lip balm, lip gloss, lip liner, eye shadow, eyeliner, mascara, nail polish, eyebrow, face powder, cheek color, hair styling agent, and hair dye.
[7] A method for producing the oily cosmetic according to any one of [1] to [6] wherein its color changes after application to the lips, skin, hair, or nails, wherein the linear or branched oil-soluble fatty acid having 16-18 carbon atoms is blended in an amount of 0.03-2.00% as a dye stabilizer.
[8] A method for controlling the coloring of the oily cosmetic according to any one of [1] to [6], wherein the linear or branched oil-soluble fatty acid having 16-18 carbon atoms is used as a dye stabilizer.

### [Advantageous Effects of the Invention]

The oil-soluble fatty acid according to the present invention can prevent lactone moiety of a fluorescein structure, which is a dye, from ring opening to form a carboxylate, so that it is possible to stably preserve an oily cosmetic which retains its initial color regardless of storage conditions and temporal change of water content of the oily cosmetic; and particularly in an oily cosmetic whose color changes after application to the skin, it is possible to prevent dark coloring before application to the skin, resulting in dark coloring after application to the skin.

### [Brief Description of Drawings]

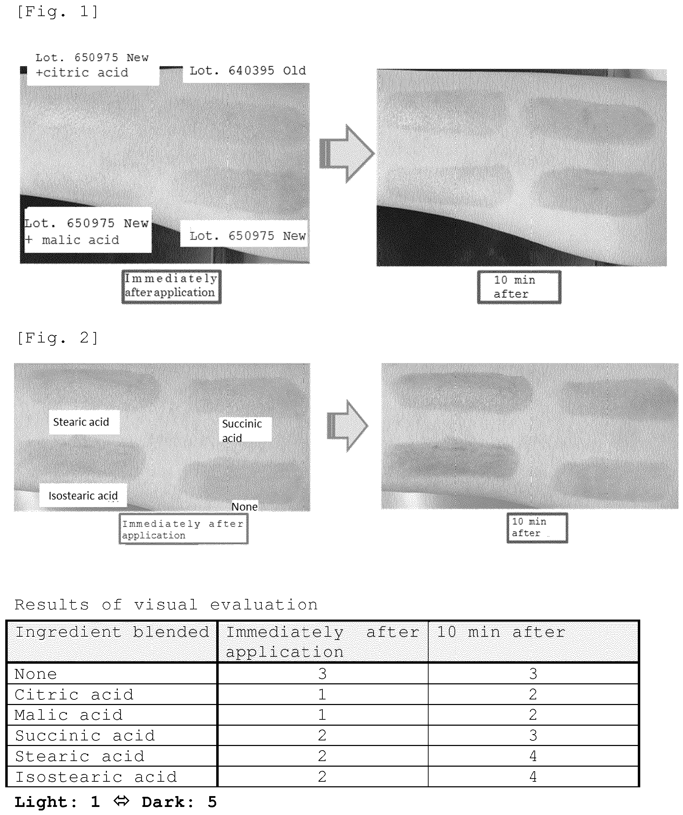

[Fig. 1] Figure 1 is a photograph showing changes in coloring immediately after application and 10 minutes after application of lipstick bases Lot 640395 and Lot 650975, as well as Lot 650975 blended with DL malic acid or citric acid at an amount of 0.1% each.
[Fig. 2] Figure 2 is a photograph showing changes in coloring immediately after application and 10 minutes after application of lipstick bases blended with isostearic acid, stearic acid, or succinic acid, at an amount of 0.1% each. Numerical values in the table in Fig. 2 are results of visual evaluation.
[Fig. 3] Figure 3 is a photograph showing changes in coloring depending on the blending amount of isostearic acid in the oily cosmetic for the lips of Example 4. Numerical values in the table in Fig. 3 are results of visual evaluation.

### [Detailed Description of the Invention]

As the linear or branched oil-soluble fatty acid having 16-18 carbon atoms of the present invention, palmitic acid, oleic acid, linoleic acid, linolenic acid, stearic acid, or isostearic acid can be used; preferably palmitic acid, isostearic acid or stearic acid, and particularly preferably isostearic acid can be used.

The blending amount of the linear or branched fatty acid in the present invention is 0.03-5.0%, preferably 0.03-2.0%, more preferably 0.03-1.0%, furthermore preferably 0.03-0.5%, particularly preferably 0.03-0.3%, and most preferably 0.03-0.1%; if it is too much, its stimulation will cause roughness of the lips, and if it is too little, storage stability and effect of changes in coloring after application to the skin cannot be obtained sufficiently.

As the oil-soluble dye of the present invention, dyes of the following CI numbers: C.I. 45350, C.I. 45370, C.I. 45425, C.I. 45410, C.I. 45440, C.I. 45100, C.I. 45170, C.I. 45380, C.I. 45430, and C.I. 45190 can be used; it is preferably C.I. 45350, C.I. 45370, C.I. 45425, C.I. 45410, C.I. 45440, C.I. 45380, C.I. 45430, and C.I. 45190, particularly preferably C.I. 45410 and C.I. 45380, and they may be used alone or in combination of two kinds or more.

In the present specification, "dye stabilizer" is an acidic agent used for adjusting the color tone of a dye in oily cosmetics.

In addition to the above oil-soluble fatty acids and oil-soluble dyes, the oily cosmetics of the present invention may contain various ingredients as necessary, for example, high-viscosity ester oil agent, low-viscosity ester oil agent, non-aqueous thickener, triester, tetraester, polyester, higher alcohol, polyamide resin, surfactant, skin conditioning agent, antioxidant, cosmetic ingredients, preservative, perfume and the like, as appropriate.

As the high-viscosity ester oil agent, the following may be used: dipentaerythrityl (hydroxystearate/stearate/rosinate), diisostearyl malate, hydrogenated castor oil isostearate, hydrogenated castor oil dimer dilinoleate, (polyglyceryl-2 isostearate/dimer dilinoleate) copolymer, (polyglyceryl-2 diisostearate/dimer dilinoleate) copolymer, (phytosteryl/isostearyl/cetyl/stearyl/behenyl) dimer dilinoleate, dimer dilinoleyl bis (phytosteryl/behenyl/isostearyl) dimer dilinoleate, di(isostearyl/phytosteryl) dimer dilinoleate, dimer dilinoleyl-hydrogenated rosin condensate, dimer dilinoleyl diisostearate, dimer dilinoleyl dimer dilinoleate, di(cholesteryl/behenyl/octyldodecyl) lauroylglutamate, di(octyldodecyl/phytosteryl/behenyl) lauroylglutamate, myristoylmethylalanine (phytosteryl/decyltetradecyl), (diglycerin/dilinoleate/hydroxystearate) copolymer and the like; preferably, it is diisostearyl malate, (polyglyceryl-2 isostearate/dimer dilinoleate) copolymer, and (polyglyceryl-2 diisostearate/dimer dilinoleate) copolymer.

As the low-viscosity ester oil agent, diisobutyl adipate, diethylhexyl succinate, cetyl ethylhexanoate, hexyldecyl ethylhexanoate, ethylhexyl isononanoate, isodecyl isononanoate, isotridecyl isononanoate, isopropyl myristate, isopropyl palmitate, isopropyl isostearate and the like can be used, and it is preferably isodecyl isononanoate.

As the nonaqueous thickener, (ethylene/acrylate) copolymer, bisdialkyl (C14-18) amide (ethylenediamine/hydrogenated dimer dilinoleate) copolymer, (VP/hexadecene) copolymer, dextrin (palmitate/ethylhexanoate), polyethylene, and microcrystalline and the like can be used, and it is preferably bisdialkyl (C14-18) amide (ethylenediamine/hydrogenated dimer dilinoleate) copolymer, dextrin (palmitate/ethylhexanoate), polyethylene, and microcrystalline.

As the triester, triethylhexanoin, trimethylolpropane triethylhexanoate, glyceryl tri(caprylate/caprate), triisostearin, trimethylolpropane triisostearate, erythrityl triethylhexanoate and the like can be used, and it is preferably glyceryl tri(caprylate/caprate).

As the tetraester, pentaerythrityl tetraethylhexanoate, pentaerythrityl tetraisostearate and the like can be used.

As the polyester, polyglycerin fatty acid esters such as polyglyceryl isostearate, polyglyceryl diisostearate, polyglyceryl triisostearate, polyglyceryl tetraisostearate and the like can be used, and it is preferably polyglyceryl isostearate.

As the higher alcohol, lauryl alcohol, myristyl alcohol, stearyl alcohol, eicosanol, behenyl alcohol, oleyl alcohol, octyl dodecanol and the like can be used.

In the oily cosmetic of the present invention, a mixture containing polyamide resin, for example, product name Haimalate PAM (produced by Kokyu Alcohol Kogyo Co., Ltd.) can be used as a base material.

As the skin conditioning agent, dibutyl lauroyl glutamide, dibutyl ethylhexanoyl glutamide and the like can be used.

Ultraviolet scattering agent is not particularly limited, and inorganic compounds such as iron oxide, cerium oxide, zirconium oxide, titanium silicate, zinc silicate, anhydrous silicic acid, cerium silicate and the like can be used.

As the antioxidant, for example, α-tocopherol, BHT, ascorbic acid, EDTA and the like can be used, and as the cosmetic ingredients, vitamins, collagen, hyaluronic acid, anti-inflammatory agents and the like can be used, and as the preservative, p-oxybenzoic acid ester, phenoxyethanol and the like can be used.

The oily cosmetic of the present invention can have various forms and product types depending on the purposes. Examples of the form include powder form, liquid form, gel form, cream form, stick form, pencil form and the like. Examples of the product type include foundation, lipstick, lip balm, lip gloss, lip liner, eye shadow, eyeliner, mascara, nail polish, eyebrow, face powder, cheek color, hair styling agent, hair dye and the like.

In the use of these ingredients, particularly preferred combinations for making transparent oily cosmetics are diisostearyl malate, bisdialkyl (C14-18) amide (ethylene diamine/hydrogenated dimer dilinoleate) copolymer.

Furthermore, preferred combinations of ingredients for obtaining the effects of the present invention are octyldodecanol, dibutyl lauroyl glutamide, dibutyl ethylhexanoyl glutamide.

### Example 1

According to the following formulation, a lip balm was produced.

**[Table 1]**

| Ingredient | Blending amount (%) |
|---|---|
| (Polyglyceryl-2 isostearate/dimer dilinoleate) copolymer | 25.00 |
| Glyceryl tri(caprylate/caprate) | 22.50 |
| Octyl dodecanol | 22.50 |
| Polyglyceryl triisostearate | 16.70 |
| Polyethylene | 10.40 |
| Microcrystalline wax | 2.60 |
| Red No. 218 | 0.10 |
| Isostearic acid | 0.10 |
| Tocopherol | 0.10 |
| Total | 100.00 |

### Example 2

According to the following formulation, a lipstick was produced.

**[Table 2]**

| Ingredient | Blending amount (%) |
|---|---|
| Isotridecyl isononanoate | 55.70 |
| Bisdialkyl (C14-18) amide (ethylenediamine/hydrogenated dimer dilinoleate) copolymer | 15.00 |
| Diisostearyl malate | 15.00 |
| Octyl dodecanol | 12.00 |
| Dibutyl lauroyl glutamide | 1.20 |
| Dibutyl ethylhexanoyl glutamide | 0.80 |
| Red No. 223 | 0.10 |
| Isostearic acid | 0.10 |
| Tocopherol | 0.10 |
| Total | 100.00 |

### Example 3

According to the following formulation, a lip gloss was produced.

**[Table 3]**

| Ingredient | Blending amount (%) |
|---|---|
| Isotridecyl isononanoate | 35.70 |
| (Polyglyceryl-2 diisostearate/dimer dilinoleate) copolymer | 20.00 |
| Diisostearyl malate | 10.00 |
| Bisdialkyl (C14-18) amide (ethylenediamine/hydrogenated dimer dilinoleate) copolymer | 1.00 |
| Dextrin (palmitate/ethylhexanoate) | 3.00 |
| Red No. 223 | 0.10 |
| Isostearic acid | 0.10 |
| Tocopherol | 0.10 |
| Total | 100.00 |

### Example 4

Blending amounts used in the experiment of changes in coloring with isostearic acid are shown in Table 1.

**[Table 4]**

| | Product name | Cosmetic ingredients labelling name | wt% |
|---|---|---|---|
| 1 | KAK 139 | Isotridecyl isononanoate | Residual amount |
| 2 | Haimalate PAM | Diisostearyl malate, Bisdialkyl (C14-18) amide (ethylenediamine/hydrogenated dimer dilinoleate) copolymer | 29.9 |
| 3 | AJK-OD2046 | Octyldodecanol, dibutyl lauroyl glutamide, dibutyl ethylhexanoyl glutamide | 15.0 |
| 4 | Red No. 218 | Red No. 218 | 0.2 |
| 5 | Isostearic acid | Isostearic acid | 0.01-0.2 |

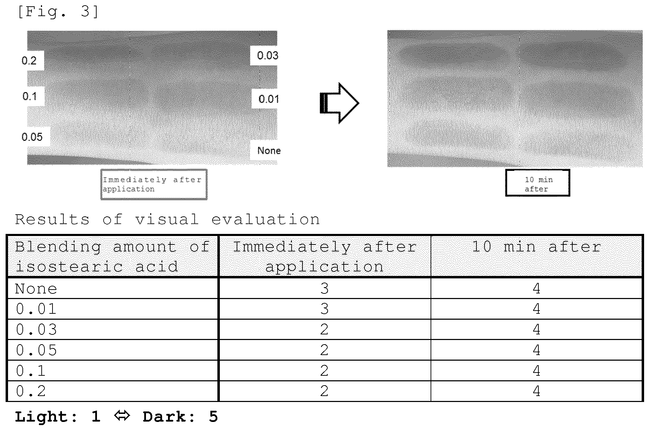

Coloring experiments were carried out with the blending amounts of isostearic acid of 0%, 0.01%, 0.03%, 0.05%, 0.1% and 0.2%, respectively; as a result, as shown in Fig. 3, when the blending amount of isostearic acid was 0.03% or more, the color immediately after application was light; and the effect of gradually darkening the color after application was observed.

## Claims

1. An oily cosmetic comprising a fluorescein dye comprising a lactone moiety, wherein the blending amount of a linear or branched oil-soluble fatty acid having 16-18 carbon atoms is 0.03-2.00%.

2. The oily cosmetic according to Claim 1, wherein the blending amount of the linear or branched oil-soluble fatty acid having 16-18 carbon atoms is 0.03-0.10%.

3. The oily cosmetic according to Claim 1 or 2, wherein the linear or branched oil-soluble fatty acid having 16-18 carbon atoms is palmitic acid, isostearic acid or stearic acid.

4. The oily cosmetic according to any one of Claims 1 to 3, wherein its color changes after application to the lips, skin, hair, or nails.

5. The oily cosmetic according to any one of Claims 1 to 4, wherein the C.I. number of the fluorescein dye comprising a lactone moiety is C.I. 45410 or C.I. 45380.

6. The oily cosmetic according to any one of Claims 1 to 5, wherein the oily cosmetic is foundation, lipstick, lip balm, lip gloss, lip liner, eye shadow, eyeliner, mascara, nail polish, eyebrow, face powder, cheek color, hair styling agent, and hair dye.

7. A method for producing an oily cosmetic according to any one of Claims 1 to 6 wherein its color changes after application to the lips, skin, hair, or nails, wherein the linear or branched oil-soluble fatty acid having 16-18 carbon atoms is blended in an amount of 0.03-2.00% as a dye stabilizer.

8. A method for controlling the coloring of the oily cosmetic according to any one of Claims 1 to 6, wherein the linear or branched oil-soluble fatty acid having 16-18 carbon atoms is used as a dye stabilizer.
